# EUROPEAN PATENT APPLICATION

(11) **EP 1 593 360 A2**
(43) Date of publication of application: **09.11.2005**
(21) Application number: 05009801.1
(22) Date of filing: 05.05.2005
(51) Int. Cl.: A61F 5/01, A43B 13/14, A43B 13/16, A61F 5/14

(54) **Footwear orthosis**

(30) Priority: 06.05.2004 IT MC20040063
(71) Applicant: Salvatelli S.r.l., 63014 Montegranaro (Ascoli Piceno) (IT)
(72) Inventor: Tulipani, Massimo, 6850 Mendrisio (IT); Salvatelli, Franco, 62012 Civitanova Marche (IT)
(74) Representative: Reniero, Cirillo Silvano

(57) **Abstract**

A footwear orthosis including a sole member (2) having a tread surface (3) and an inner surface (5), and a partly open upper member (4) designed to be anchored to the sole member and arranged removably to secure a user's foot thereto, the tread surface (3) being substantially uninterruptedly flat and at least partly outwardly convex.

## Description

The present invention relates to a footwear orthosis for a user having problems with his foot, e.g. following a surgical operation or owing to a foot disease.

A number of people after undergoing a surgical operation in one or both their feet have to use a footwear orthosis, I. e. a specifically structured footwear, suitable for making it possible for the user to walk with a reasonable comfort without damaging or stressing specific sensitive foot portions. More particularly, when a patient is affected by insulin-dependant diabetes some foot tissues degenerate and diabetic ulcers are formed, and when ulceration occurs the patient cannot tolerate any exerted pressure on the foot.

Searches have been conducted to devise a footwear orthosis that makes it possible for a patient with one or both ill feet to walk without aggravating or otherwise compromising the health state of his foot or feet.

The footwear orthoses used up to now usually have an upper mounted on a sole having a front or rear raised (thicker) flat portion, a lower flat portion, and an intermediate portion which is inwardly convex. Such a configuration allows the patient's footwear to avoid during walking contact with the ground, and thus pressure transmitted to the foot, at the lower and intermediate portions thereof where his most sensitive foot parts are located.

Such a solution, although being satisfactory from some viewpoints, prevents the patient from walking naturally, as the patient while walking has to lift the orthosis and touch down at the raised portion of the footwear, i. e. with a trim substantially normal to the soil, otherwise the orthosis must be set in an inclined trim in order to touch the soil at the orthosis tip. In both cases the resulting movements of the patient's foot are quite unnatural and uncomfortable, and will slow down the patient's advancing movement.

The main object of the present invention is to provide a footwear orthosis which prevents its user from negatively affecting a specific sensitive portion of his foot or feet, and makes it possible for him or her to walk naturally at the same time.

Another object of the present invention is to provide a footwear orthosis designed to supports the user's foot in a suitable walking trim and to be used for different rehabilitation stages of a patient's foot.

Another object of the present invention is to provide a footwear orthosis which is can be produced at competitive costs so as to be advantageous also from an economical point of view.

These and other objects that will better appear below are achieved by a footwear orthosis including a sole member having a tread surface and an inner surface, and a partly open upper member designed to be anchored to said sole member and arranged removably to secure a user's foot thereto, characterized in that said tread surface is substantially uninterruptedly flat and at least partly outwardly convex.

Advantageously, the footwear orthosis comprises at least one intermediate flat portion and at least one front and/or rear portion outwardly convex, said front and/or rear convex portion allowing, in use, said sole member at least partly to roll between an Initial ground touching position and a lifting position in which its front portion is leaving the ground.

According to another aspect of the present invention there is provided an insole element for a footwear orthosis comprising at least two portions engageable with one another in a puzzle-like manner.

Further features and advantages of a footwear orthosis according to the present invention will better appear from the following detailed description of some presently preferred embodiments thereof, given by way of non-limiting examples of carrying out the invention, with reference to the accompanying drawings, in which:
Figure 1 shows a perspective view with parts cut away of a footwear orthosis according to the present invention;
Figure 2 illustrates a side view of the lower portion of the footwear orthosis of Fig. 1;
Figure 3 is a bottom view of the footwear orthosis of Fig. 1;
Figures 4 and 5 are a bottom and side view, respectively, of an insole reinforcing member according to the present invention;
Figure 6 is a cross-section view taken along the line VI-VI of the insole reinforcing member in Fig. 5;
Figure 7 is a cross-section view taken along the line VII-VII of the insole reinforcing member in Fig. 5;
Figure 8 is a top view of an assembled puzzle-like insole reinforcing member;
Figure 9 shows a perspective view of a reinforcing insole member of Fig. 8 with three portions thereof reciprocally engaged in a puzzle-like manner; and
Figure 10 is a perspective view in explosion of two portions of the insole reinforcing member of Fig. 9.

in the accompanying drawings, the same or similar parts or components have been indicated with the same reference numerals.

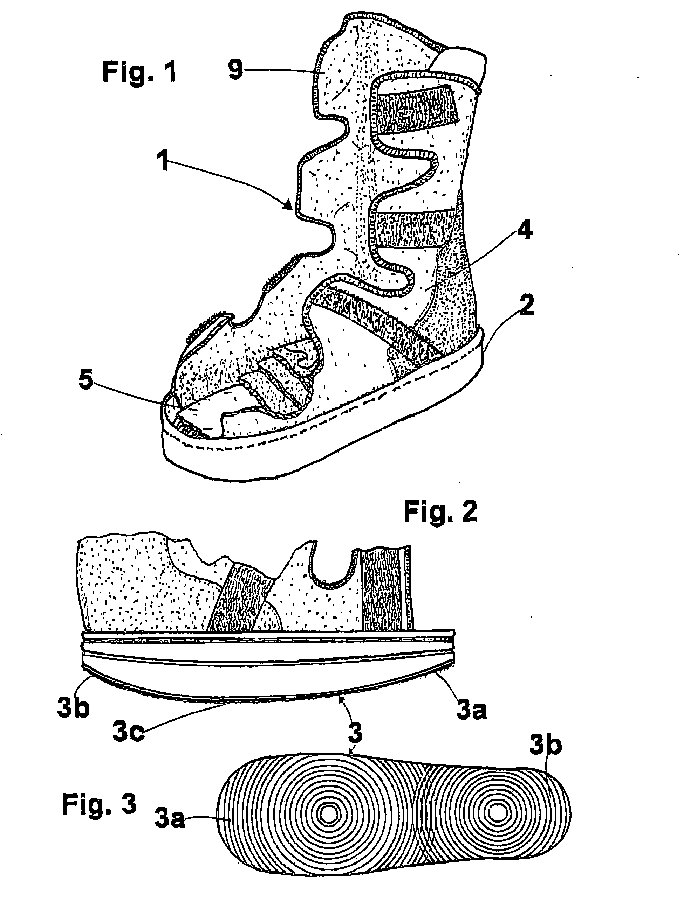

With reference first to Figures 1 to 3, it will be noted that a footwear orthosis 1 has an ambidextrous sole 2 with a tread surface 3, and an upper 4 comprising a bootleg portion.

The tread surface 3 is substantially uninterruptedly flat, i. e. a continuous flat surface substantially with no bulging or recessed portions, that can be imagined as obtained from a straight generatrix moving along a path including straight and/or at least one curved portion that give rise to a corresponding number of outwardly convex tread portions.

Of course, the tread surface 3 can include slightly in relief tread formations such as those generally indicated as two concentric groups of circles in Fig. 3, for proper grip with the ground.

The tread outwardly convex portion or portions of tread surface 3, preferably have a variable radius of convexity. More particularly, the tread surface has a front portion 3a and rear portion 3b which are outwardly (downwardly, in use) convex, having a radius of convexity larger than that at the intermediate portion 3c.

The sole 2 has Its upper (inner) surface 5 which is substantially flat and structured as a lattice as it is well known to a person skilled in the art. A peripheral edge 6 extends upwardly from the upper surface 5 and substantially perpendicularly to the inner surface 5, the edge 6 having a height preferably of about 45 mm.

On the upper surface a relatively rigid intermerdiate insole member 7 is provided which preferably has an, in use, upper flat surface, while its lower surface in contact with the upper surface 5 of the sole has a lattice-like rib structure 7a so dimensioned as to be set in position without clearance on the lattice of the upper surface 5. The intermediate insole member 7 is preferably made of moulded antibacterical and washable ABS (acrilvnitrile-butadiene-stirene) material.

A standard insole, usually bearing a trade mark, a design or the like, could be provided on the insole member 7.

The footwear orthosis shown in Figure 1 has a suitably shaped upper 4 with a bootleg portion and a number (four) of suitable securing means 9, e. g. each comprising a ribbon provided with an anchoring means such as a fastening means, e.g. a Velcro strip, arranged removably to secure a patient's foot to the footwear. Such a footwear orthosis makes it possible to leave the instep or upper portion of the user's foot loose and practically free from footwear portions that might exercise a undue pressure on the user's foot while walking.

During walking the user lower the footwear orthosis at its rear portion 3b having a relatively large radius of curvature which makes it possible for the sole to effect a soft progressive rolling in the forward direction with no abrupt (torsional) stress being transmitted to the user's foot. The user's foot makes then a smooth swing movement while the footwear sole keeps in contact with the ground throughout its intermediate portion 3c and part of its front portion 3a. Once owing to rolling action the front portion 3a comes into contact with the ground the user foot is raised for a new step forward and so on.

It should be emphasized that, as an important difference with footwear orthoses according to the prior art, with a footwear orthosis according to the present invention the user touches the ground with the rear convex portion 3b of the footwear, makes a rolling movement across the intermediate portion 3c, and raises the footwear only after or upon the front convex portion 3a being rolled into contact with the ground.

The specific structure of the footwear orfihosis according to the present invention thus prevents dangerous pressure picks being applied to the user's foot while the sole 2 is touching down or leaving or taking off the ground.

The touching down step is soft operation even because the rear portion 3b is then the footwear portion nearer to the ground, the user's foot being thrown forwards with its tip portion extending upwards, and thus the curved (convex) surface of the rear portion prevents the sole 2 from abruptly touching down the ground.

The same applies to lifting or take off step that takes place in an gradual and smooth way owing to the curved (convex) configuration of the front portion 3a of the sole 2.

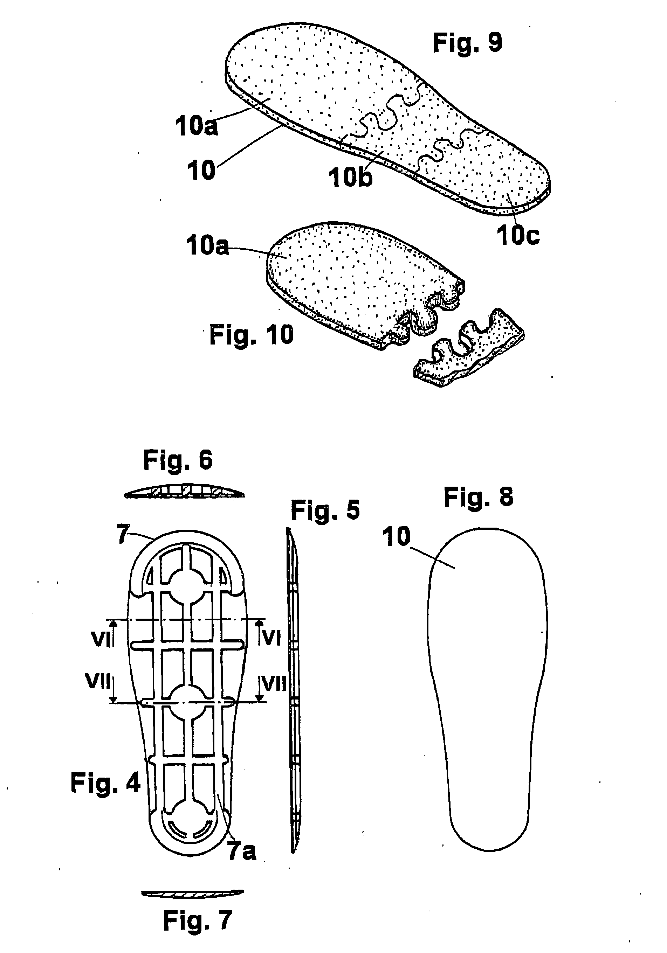

With reference now to Figure 4 and 5, an insole 10 is shown which comprises three portions 10a, 10b and 10c that can be removably connected together in a puzzle-like manner.

The insole 10 is preferably made of a cushioning material and more specifically an antibacteric, washable cushioning material.

An insole 10 can include a number (typically two or three) portions 10a made of different materials thereby obtaining an insole element having mechanical characteristics changing from its rear to its front portion. Such an insole makes it possible to adapt a footwear orthosis according to the invention to specific user's needs.

Preferably, the peripheral edge 6 of the sole 2 can be dimensioned so as to make it possible to locate three or more superimposed insoles 10 in the footwear orthosis.

The footwear orthosis as above described is susceptible to numerous modifications and variations within the scope as defined by the claims.

Thus the insole can comprises portion 10a to 10c formed with an aperture or through opening to be located at sensitive portion of the user's foot.

## Claims

1. A footwear orthosis including a sole member (2) having a tread surface (3) and an inner surface (5), and a partly open upper member (4) designed to be anchored to said sole member and arranged removably to secure a user's foot thereto, **characterized in that** said tread surface (3) is substantially uninterruptedly flat and at least partly outwardly convex.

2. A footwear orthosis as claimed in claim 1, **characterized in that** said tread surface (3) comprises at least one intermediate flat portion (3c) and at least one outwardly convex front (3a) and/or rear portion (3b), whereby said tread surface (3) can roll on the ground between a touch down position in which said rear portion (3b) comes into contact with the ground through out said intermediate portion, and a take off position at said front convex portion (3a).

3. A footwear orthosis as claimed in claim 1 or 2, **characterized in that** it comprises an intermediate insole (7) reinforcing member locatable on said inner surface of said sole member.

4. A footwear orthosis as claimed in claim 3, **characterized in that** said intermediate insole (7) has a lattice structure (7a) at the surface thereof designed to contact said inner surface (5) of said sole member.

5. A footwear orthosis as claimed in claim 3 or 4, **characterized in that** said intermediate insole (7) is obtained by moulding a relatively stiff or rigid material.

6. A footwear orthosis as claimed in claim 5, **characterized in that** said relatively stiff or rigid material is antibacterial acrylonitrile butadiene styrene.

7. A footwear orthosis as claimed in any previous claim, **characterized in that** said upper member (4) comprises removable securing means to a user's foot.

8. An insole element (10) for a footwear orthosis **characterized in that** comprises at least two portions (10a, 10b, 10c) mutually engageable in a puzzle-like manner.

9. An insole element as claimed in claim 8, **characterized in that** each portion (10a, 10b, 10c) has a different density value.

10. An insole element as claimed in claim 8 or 9, **characterized in that** each portion (10a, 10b, 10c) is made of suitable plastics material.
